# FASCICULE DE BREVET EUROPEEN

(11) **EP 0 470 144 B1**
(45) Date de publication et mention de la délivrance du brevet: **03.05.1995**
(21) Numéro de dépôt: 90907159.9
(22) Date de dépôt: 27.04.1990
(51) Int. Cl.: A61K 31/195

(54) **D-ACIDE ASPARTIQUE BETA-HYDROXAMATE POUR LE TRAITEMENT DES INFECTIONS VIRALES ET DES TUMEURS**
D-ASPARAGINSÄURE-BETA-HYDROXAMAT ZUR BEHANDLUNG VIRALER INFEKTIONEN UND TUMOREN
BETA-HYDROXAMATE D-ASPARTIC ACID FOR TREATMENT OF VIRAL INFECTIONS AND TUMOURS

(30) Priorité: 28.04.1989 FR 8905744
(43) Date de publication de la demande: 12.02.1992
(73) Titulaire: INSTITUT NATIONAL DE LA SANTE ET DE LA RECHERCHE MEDICALE (INSERM), 75654 Paris Cédex 13 (FR)
(72) Inventeur: VILA, Jorge, F-69540 Irigny (FR); THOMASSET, Nicole, F-69100 Villeurbanne (FR); HAMEDI SANGSARI, Farid, F-69009 Lyon (FR); GRANGE, Jacques, F-69600 Oullins (FR)
(74) Mandataire: Guerre, Dominique
(86) Numéro de dépôt international: FR9000307
(87) Numéro de publication internationale: WO9013291

(56) Documents cités:
- EP-A- 250 335
- Cancer Chemotherapy Report (1972), vol 3(2), p.24-57
- Archs Biochem. Biophys (1969), vol 129, p.560-566
- Neurochem.Int., vol,10,no.2, 1987,Pergamon Journal Ltd.,(GB) V.J. Balcar et al: " Differences between substrate specificities of L-glutamate uptake by neurons and glia, studied in cell lines and primary cultures", pages 213-217
- Biochem. Pharmacol., vol.26, no.15, 1977, J.R.Uren et al.: " Effects of asparagine synthetase inhibitors on asparaginase resistant tumors",pages 1405-1410
- Chemical Abstracts,vol. 75, 1971,(Columbus, Ohio, US)voir page 281, resume 40401m & JP,A,7108683 (Tanabe Seiyaku Co., Ltd) 5 mars 1971
- "Progress in Antimicrobiol. and Anticancer Chemotherapy" vol. 2 1970 M. Miura et al."Antitumor activity of..." pages 170-174.

## Description

La présente invention concerne l'application en tant que médicament du D-acide aspartique β-hydroxamate, en abrégé DAH, de formule : 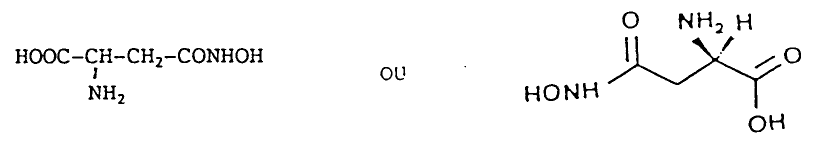
Le DAH peut être formé par la réaction d'un groupe acyle activé de l'isomère D de l'acide aspartique ou d'un de ses dérivés, avec l'hydroxylamine suivant la réaction : 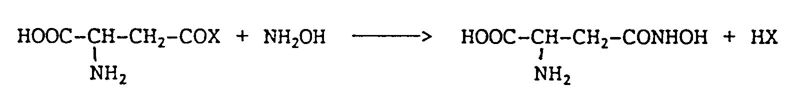
- COX étant un groupement acide, amide, ester ou anhydride.

Le DAH peut aussi être préparé par voie enzymatique, à partir de l'acide D-aspartique. Il s'agit d'un produit connu disponible sur le marché, par exemple le produit A 9009 de la Société SIGMA dans le catalogue 1987 (version française).

Suivant un procédé avantageux et préféré, le DAH est préparé par hydroxy-amination de la D-asparagine ou du D-acide aspartique β-éthyl-ester, celui-ci étant obtenu par la mono-estérification de l'acide D-aspartique, comine le montre la séquence réactionnelle ci-dessous : 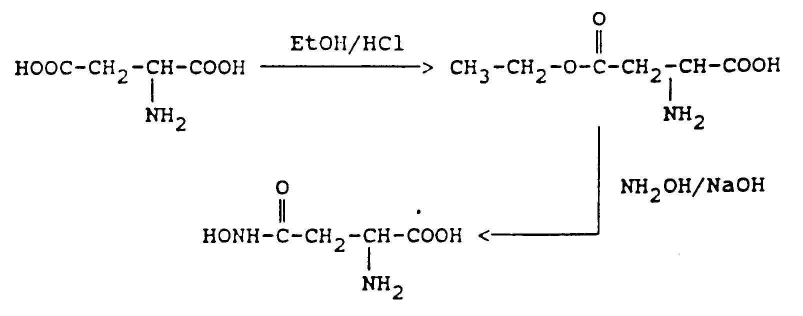
La caractérisation chimique du DAH résulte d'abord de son analyse élémentaire qui est la suivante :
Formule brute : C₄H₈N₂O₄
Poids moléculaire : 148,1
C = 32,56 % - H = 5,5 % - N = 18,6 % - O = 41,98 %
Le DAH est encore caractérisé par les analyses spectrales ci-après :

### Analyse en résonance magnétique nucléaire (RMN) :

Le DAH a été analysé en spectre RMN ¹³C et ¹H, respectivement à 50,4 MHz et 200 MHz sur un appareil Brucker avec transformée de Fourier, dans du solvant D₂O. Les déplacements chimiques correspondant aux différents signaux ont été exprimés en ppm par rapport au tétraméthylsilane (TMS) pris comme référence externe.

Le spectre de RMN ¹³C comporte quatre signaux caractéristiques correspondant respectivement :

| | |
|---|---|
| - a la fonction acide (COOH) : | 172,8 ppm |
| - a la fonction hydroxamate (CONHOH) : | 168,4 ppm |
| - au carbone portant la fonction NH₂ : | 51,1 ppm |
| - au carbone portant la fonction CH₂ : | 32,2 ppm |

Dans le spectre de RMN ¹H, les trois protons en position α et β du DAH constituent un système de type ABX. Hα est couplé avec Hβ (J = 7,5 Hz) et chacun des deux pics ainsi obtenus est à nouveau dédoublé par Hβ' (J = 4,5 Hz) et on obtient un quadruplet à 4,2 ppm. Hβ' voit son pic dédoublé par Hβ (J = 16 Hz) puis par Hα (J = 4,5 Hz) pour donner un quadruplet à 3,0 ppm.

De la même façon Hβ est couplé avec Hβ' (J = 16 Hz) et avec Hα (J = 7,5 Hz) pour former un quadruplet à 2,9 ppm.

Les protons fixés sur les atomes d'oxygène et d'azote sont échangés avec le D20 et leurs signaux se trouvent à 5 ppm dans le pic (HOD).

### Spectrométrie de masse :

Le spectre de masse du DAH a été établi sur un VARIAN MAT CHS avec une énergie d'ionisation de 70 eV. L'ion moléculaire du DAH a une abondance relative nulle.
- m/e (%) :: M⁺148 (O); 97 (11); 97 (37); 54 (35); 44 (100); 43 (91); 42 (43); 41 (29); 28 (75); 27 (29); 26 (80); 18 (42); 17 (23).

### Spectrophotométrie :

La fonction hydroxamate du DAH est mise en évidence par un complexe Fe3+-hydroxamate qui donne une coloration rouge-violet, quantifiée par méthode colorimétrique à 550 nm, à l'aide d'un spectrophotomètre de type Beckman DU 70.

### Stabilité du DAH :

La stabilité du DAH en fonction du pH et de la température a été déterminée sur une durée d'un mois par dosage colorimétrique spécifique de la fonction hydroxamate. Les résultats du tableau ci-après montrent que dans l'eau le DAH est stable jusqu'à une température de 50°C. Une dégradation significative du produit n'est constatée que dans des conditions extrêmes de pH, comme l'illustre le Tableau 1 ci-après.

**Tableau 1**

| Pourcentage de dégradation du DAH sur une période de 30 jours selon le pH et la température. | | | |
|---|---|---|---|
| Température (°C) | NaOH 0,1 N | H₂O | HCl 0,1 N |
| + 4 | 4 % | 0 % | 42 % |
| + 25 | 36 % | 0 % | 78 % |
| + 50 | 100 % | 0 % | 100 % |

L'invention a pour objet l'application du DAH à titre de médicament, en particulier destiné au traitement des infections virales, plus particulièrement celles dues aux rétrovirus, notamment ceux provoquant le SIDA, et au traitement des tumeurs.

Dans la demande de brevet EP-0 250 335, il a été montré que le DAH potentialisait l'activité antitumorale de la L-asparaginase. Il est connu en effet que la L-asparaginase est un agent antitumoral. Cette activité s'avérant insuffisante, on a essayé de la renforcer en associant la L-asparaginase il d'autres produits. L'association avec le DAH n'a pas procuré de résultats exploitables pratiquement.

Conformément à la publication Biochem. Pharmacol., Vol 26, Nr 15, 1971, pages 1405-1410, on a décrit l'activité antitumorale de certains inhibiteurs d'asparagine synthéthase sur les tumeurs résistantes à l'asparaginase, au rang desquels figurent le mélange racémique D,L-β aspartylhydroxamate. L'activité antitumorale du racémique est attribuée à l'isomère LAH.

### Conformément à la publication suivante :

"Differences between substrate specificities of L-Glutamate uptake by neurons and glia, studied in cell lines and primary cultures" Vladimir J. Balcar et al, Neurochem. Int., Vol. 10, No 2, 1987, pages 213-217, Pergamon Journals Ltd
on a étudié la physiologie du passage du L-glutamate dans des cellules tumorales, en l'occurence des cellules gliomateuses, et l'effet du DAH et du LAH sur cette absorption du L-glutamate. Selon ce document, les auteurs ont mis en évidence l'inhibition de l'absorption du L-glutamate, à l'exclusion de toute activité antitumorale du DAH ou LAH.

On a trouvé maintenant de façon surprenante que le DAH seul avait une activité anti-rétrovirale, en particulier contre les virus responsables du SIDA. On a trouvé également que le DAH, administré à des dosages spécifiques, possédait une action inhibitrice sur les tumeurs.

Il est bien connu que, en dépit des progrès accomplis en ce qui concerne la lutte contre les infections virales, on ne dispose pas encore d'une gamme d'agents antiviraux aussi étendue, sûre et efficace que la gamme des antibiotiques, par exemple.

En conséquence, la présente invention concerne :
- un médicament caractérisé en ce qu'il comprend du D-acide aspartique β monohydroxamate (DAH), comme agent thérapeutique, dans une quantité thérapeutiquement active, à l'exclusion de toute composition médicamenteuse dans laquelle le DAH est, soit présent dans une quantité subthérapeutique, soit en combinaison avec la L-asparaginase; et en particulier un médicament dans lequel le DAH est le seul agent thérapeutique
- l'utilisation du DAH pour l'obtention d'un médicament, à l'exclusion de toute utilisation du DAH pour l'obtention d'une composition médicamenteuse dans laquelle le DAH est, soit présent dans une quantité subthérapeutique, soit en combinaison avec la L-asparaginase
- l'utilisation d'une composition comprenant le DAH, pour l'obtention d'un médicament, à l'exclusion de l'utilisation de toute composition dans laquelle le DAH est, soit présent dans une quantité subthérapeutique, soit en combinaison avec la L-asparaginase
- l'utilisation du DAH ou d'une composition comprenant du DAH, à l'exclusion de toute composition-dans laquelle le DAH est, soit présent dans une quantité subthérapeutique, soit en combinaison avec la L-asparaginase, ceci pour l'obtention d'un médicament antiviral, et plus particulièrement un médicament anti-rétroviral, et notamment comme agent contre les virus du SIDA.

De par leur mode d'action, les agents antiviraux peuvent être classés d'une façon large, en trois catégories, les frontières entre elles étant souvent arbitraires : les agents chimiques, les stimulateurs du système immunitaire et les activateurs de l'état cellulaire antiviral.

Les agents chimiques sont les plus répandus, mais leur spectre d'action est généralement très limité et ils finissent souvent par sélectionner des variants viraux résistants, de la même manière que le font les antibiotiques avec les bactéries (DE CLERCQ E., Biochem. J., 206, 1-13, 1982 ; BECKER Y. et HADAR J., Prog. Med. Virol., 26, 1-44, 1980).

A part les différents types de vaccins, les activateurs de la réponse immune sont des agents capables d'activer les défenses non spécifiques (macrophages, cellules "tueuses" naturelles) ou, plus rarement, les défenses spécifiques (lymphocytes "B" ou "T") de l'organisme. Pour la plupart, ces agents sont encore en phase expérimentale. Contrairement aux agents chimiques, les caractéristiques essentielles de leur mode d'action sont leur spectre d'action très large et le fait qu'ils ne peuvent pas sélectionner de variants résistants. Mais ils sont pour l'instant en nombre limité et peu efficaces, bien que des progrès aient été récemmenta accomplis dans ce sens (KOFF W.C., SHOWALTER S.D., HAMPAR B. et FIDLER I.J., Science, 228, 495-496, 1985).

Les inducteurs de l'état cellulaire antiviral comprennent l'interféron (IFN), les inducteurs d'IFN et les substituts d'IFN. Il s'agit d'un groupe encore très réduit de substances qui se caractérisent pour éveiller dans les cellules un état de résistance à l'infection virale. Ces agents cumulent une puissance de l'action au niveau cellulaire des agents chimiques et le spectre d'action très large des activeurs du système immunitaire. Du fait qu'ils agissent sur toutes les cellules, qu'elles aient été infectées ou non, un élément déterminant de leur efficacité est leur faible pouvoir toxique envers les cellules.

Parmi les virus, les rétrovirus représentent un groupe responsable d'affections particulièrement graves et difficiles à combattre.

Les rétrovirus et leur pouvoir cancérigène ne sont pas une nouveauté scientifique. Dès le début du siècle, plusieurs chercheurs avaient identifié, chez l'animal, des agents transmissibles susceptibles de causer des leucémies et des tumeurs solides (ROUS P., J. Am. Med. Assoc., 56, 198, 1911). Au cours des décennies suivantes, on a trouvé des rétrovirus chez de nombreuses espèces animales. Les efforts prolongés de chercheurs amenèrent l'isolement, en 1980, du premier rétrovirus humain : le virus lymphotrope des lymphocytes T humains de type I (HTLV-I) (POIESZ B.J. et al, Proc. Natl Acad. Sci. USA, 77, 7415, 1980). Le HTLV-I engendre un cancer, rare et extrêmement malin, la leucémie à lymphocytes T de l'adulte, qui est endémique dans certaines régions du Japon, de l'Afrique et des Caraïbes.

Une autre affection très grave et importante causée par des rétrovirus, est le SIDA.

Les premiers cas de SIDA furent diagnostiqués en 1981 (GOTTREB, M.S. et al, Weekly Record, 30, 250, 1981). L'agent responsable : le virus de l'immunodéficience humaine (HIV), un rétrovirus, fut découvert en 1983 (BARRE SINOUSSI F. et al., Science, 220, 868, 1983). On recherche donc actuellement des agents actifs contre ce rétrovirus. Devant les problèmes que pose la mise au point d'un vaccin dans un bref avenir, les recherches du produit pouvant inhiber la réplication de ce rétrovirus sont nécessaires.

Les études expérimentales concernant les antiviraux portent fréquemment sur la maladie induite par le virus de Friend.

Le virus FLV est un rétrovirus murin de type C qui produit des effets rapides sur l'érythropoïèse, les précurseurs érythroïdes étant les cellules cibles de ce virus. Le complexe de Friend comprend un virus transformant défectif pour la réplication appelé SFFV (Spleen Focus Forming Virus), car il est capable d'induire des foyers macroscopiques de cellules transformées dans la rate de la souris adulte infectée et d'un virus helper appelé le F-MuLV (Friend Murine Leukemia Virus) qui sert à la réplication. Il existe deux variants de ce virus : le FV-A qui induit une anémie et le FV-P qui produit une polycytémie. L'érythroleucémie induite par le FL-V montre deux étapes : un stade précoce caractérisé par la croissance rapide du nombre des précurseurs érythropoïétiques relativement matures avec une capacité de prolifération limitée, et un stade tardif caractérisé par l'émergence de précurseurs indifférenciés avec une capacité de prolifération intense. In vivo, les manifestations de cette maladie sont une splénomégalie accompagnée d'une hépatomégalie, d'une hyperplasie érythroïde dans la rate et la moëlle osseuse, d'une polycytémie et de foci macroscopiques dans la rate. Les dernières étapes sont caractérisées par la présence de cellules érythroïdes immortelles et transplantables.

Selon l'invention, on a montré que le DAH provoque une importante inhibition de la multiplication de virus appartenant à la famille des rétrovirus ou des virus dont la réplication s'y apparente, tels que les virus dits "hepadna viridae". Ceci a été démontré in vitro avec le virus du SIDA (HIV) et in vivo avec le virus de FRIEND (FLV - Friend Leukemia Virus) qui est un rétrovirus murin de type C.

Les rétrovirus, comme tout autre virus, ne peuvent se reproduire sans détourner à leur profit l'appareil biosynthétique de la cellule. La particularité réside dans leur aptitude à inverser le courant habituel de l'information génétique. Dans les rétrovirus, le matériel génétique est constitué d'ARN, qu'une enzyme, la transcriptase inverse, utilise pour synthétiser de l'ADN ; cet ADN viral peut s'intégrer au génome de la cellule hôte.

La présente invention a également pour objet une nouvelle composition médicamenteuse, destinée notamment au traitement des infections virales, en particulier celles liées au SIDA, ainsi qu'au traitement des tumeurs, caractérisée par le fait qu'elle renferme, à titre de principe actif, dans un support pharmaceutiquement acceptable le D-acide aspartique β-monohydroxamate (DAH). La composition de l'invention peut également contenir des additifs inertes ou pharmacodynamiquement actifs.

La composition médicamenteuse de l'invention peut se présenter sous la forme d'une poudre lyophilisée de la substance active à dissoudre extemporanément dans une solution physiologique, en vue de l'injection. Le médicament peut alors s'administrer par voie parentérale, par exemple intraveineuse, intrapéritonéale, dans le liquide céphalo-rachidien, etc. Pour l'injection, le principe actif est dissous dans une solution physiologique jusqu'à obtention de la concentration désirée pour l'administration.

La composition médicamenteuse selon l'invention peut également se présenter sous une forme appropriée pour l'administration par voie orale. Par exemple, les formes appropriées sont les comprimés, les gélules, les dragées, les poudres et les granulés.

Concernant la posologie du médicament selon l'invention, on indiquera que les doses à administrer sont variables selon la durée du traitement, la fréquence de la prise, l'hôte, la nature et la gravité de la maladie. On peut par exemple mentionner l'administration d'une quantité supérieure à 1 g et pouvant aller jusqu'à quelques grammes de substance active sur une période échelonnée et limitée ainsi qu'il ressortira de la description détaillée ci-après.

Le D-acide aspartique β monohydroxamate (DAH) peut être préparé par monoestérification de l'acide D-aspartique en vue de l'obtention du D-acide aspartique β-éthyl ester, et par l'hydroxylation de ce dernier, ce qui conduit au DAH. Le DAH peut être aussi préparé par hydroxy-amination de la D-asparagine.

Les médicaments selon la présente invention peuvent être préparés et obtenus sous différentes formes :
- toute forme adaptée à une administration par voie orale, comprenant un support pharmaceutiquement acceptable du DAH
- toute forme adaptée à une administration parentérale, notamment par voie veineuse périphérique.

### EFFET DU DAH SUR LA REPLICATION DU HIV

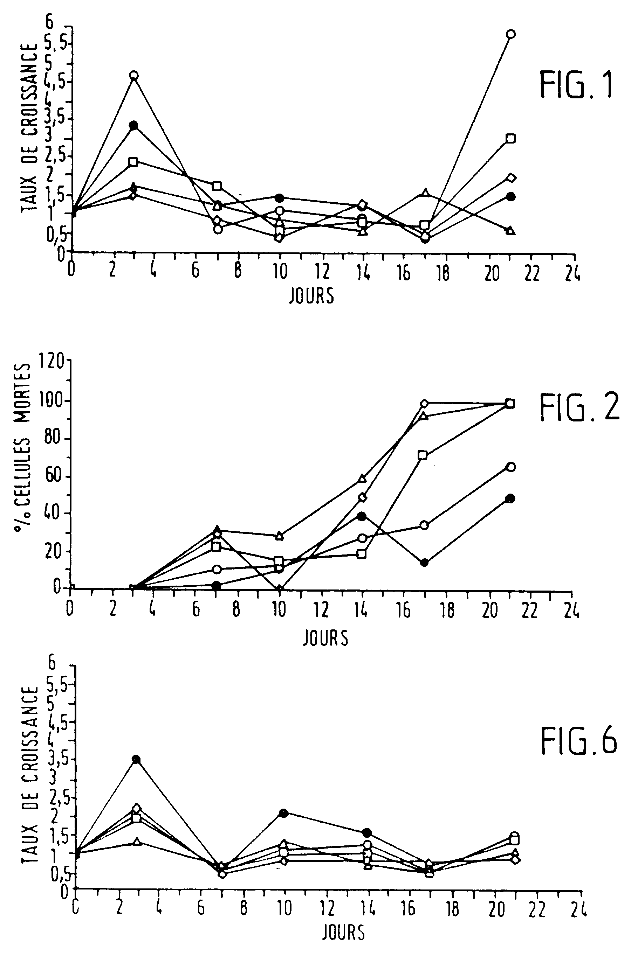

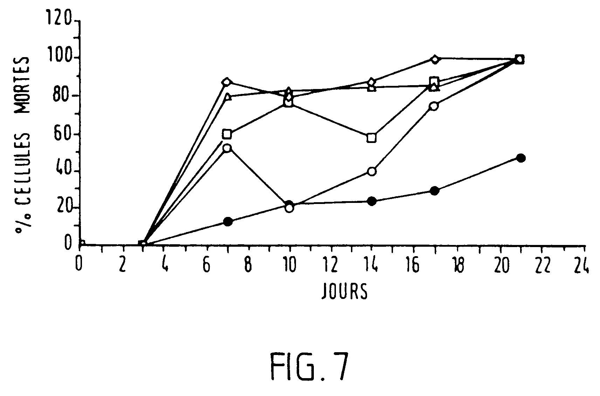

Selon l'invention, on a montré que le DAH inhibe de manière totale la multiplication virale du HIV. Cette inhibition est dose-dépendante. Les essais ont d'abord porté sur des lymphoytes de donneurs sains (Figures 1 et 2). On a pu mettre en évidence cette activité sur deux modèles de réplication du HIV : a) lymphocytes normaux infectés par le virus purifié et b) coculture des lymphocytes des malades du SIDA et de lymphocytes normaux. On a mesuré l'activité transcriptase inverse pendant 22 jours dans le surnageant de cultures cellulaires comme critère de prolifération virale. Le traitement avec le DAH a duré 72 heures de J₀ à J₃ de la manipulation (Figures 3, 4 et 5).
On a constaté que les lymphocytes ont proliféré pendant le traitement avec le produit, que ce produit n'est pas toxique pour les lymphocytes non infectés par le virus HIV (Figures 1 et 2). Par contre, le DAH s'est montré plus toxique vis-à-vis des lymphocytes infectés par le virus du SIDA (Figures 6 et 7).

### Légende de la Figure 1

### Effet du DAH sur le taux de croissance de lymphocytes de donneurs sains.

(●) absence de DAH
(o) concentration en DAH = 0,5 mM
(□) concentration en DAH = 1 mM
(△) concentration en DAH = 1,5 mM
(◇) concentration en DAH = 2 mM
. En abscisses : temps de culture (en jours)
. En ordonnées : taux de croissance

### Légende de la Figure 2

### Effet du DAH sur la mortalité cellulaire de lymphocytes sains.

(●) absence de DAH
(o) concentration en DAH = 0,5 mM
(□) concentration en DAH = 1 mM
(△) concentration en DAH = 1,5 mM
(◇) concentration en DAH = 2 mM
. En abscisses : temps de culture (en jours)
. En ordonnées : proportion de cellules mortes (en %)

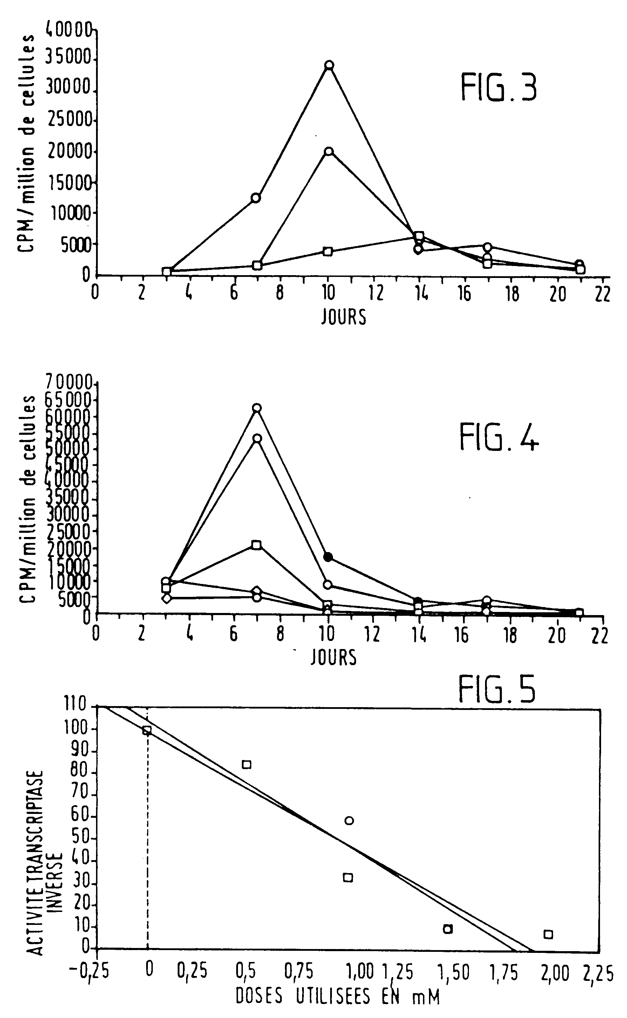

### Légende de la Figure 3

### Effet du traitement par le DAH durant trois jours sur la production de transcriptase inverse virale par une coculture de lymphocytes des malades du SIDA et de lymphocytes normaux.

(●) absence de DAH (témoin)
(o) concentration en DAH = 1 mM
(□) concentration en DAH = 1,5 mM
. En abscisses : temps de croissance des cultures après inoculation du DAH (en jours)
. En ordonnées : activité transcriptase inverse (en CPM/million de cellules)

### Légende de la Figure 4

### Effet du traitement par le DAH durant trois jours, sur la production de transcriptase inverse virale d'une culture de lymphocytes infectés par du virus purifié.

(●) absence de DAH (témoin)
(o) concentration en DAH = 0,5 mM
(□) concentration en DAH = 1,0 mM
(△) concentration en DAH = 1,5 mM
(◇) concentration en DAH = 2,0 mM
. En abscisses : temps de croissance des cultures après inoculation du DAH (en jours)
. En ordonnées : activité transcriptase inverse (en CPM/million de cellules)

### Légende la Figure 5

### Effet dose sur le maximum du pic d'activité de la transcriptase inverse.

(o) coculture
(□) culture infectée par du virus purifié
. En abscisses : doses utilisées (en mM)
. En ordonnées : activité transcriptase inverse.

### Légende de la Figure 6

### Effet du DAH sur le taux de croissance de lymphocytes infectés.

(●) absence de DAH
(o) concentration en DAH = 0,5 mM
(□) concentration en DAH = 1 mM
(△) concentration en DAH = 1,5 mM
(◇) concentration en DAH = 2 mM
. En abscisses : temps de culture (en jours)
. En ordonnées : taux de croissance.

### Légende de la Figure 7

### Effet du DAH sur la mortalité cellulaire de lymphocytes infectés.

(●) absence de DAH
(o) concentration en DAH = 0,5 mM
(□) concentration en DAH = 1 mM
(△) concentration en DAH = 1,5 mM
(◇) concentration en DAH = 2 mM
. En abscisses : temps de culture (en jours)
. En ordonnées : proportion de cellules mortes (en %).

### EFFET DU DAH SUR LA MALADIE INDUITE PAR LE VIRUS DE FRIEND.

Le virus FLV est un rétrovirus murin de type C qui produit des effets rapides sur l'érythropoïèse, les précurseurs érythroïdes étant les cellules cibles de ce virus. Le complexe de Friend comprend un virus transformant défectif pour la réplication, appelé le SFFV (Spleen focus forming virus) car il est capable d'induire des foyers macroscopiques de cellules transformées dans la rate de la souris adulte infectée, et d'un virus helper appelé le F-MuLV (Friend Murine Leukemia Virus) qui sert a la réplication. Il existe deux variants de ce virus : le FV-A qui induit une anémie et le FV-P sur lequel ont été effectués les travaux et qui produit une polycytémie. L'érythro-leucémie induite par le FL-V montre deux étapes : un stade précoce caractérisé par la croissance rapide du nombre des précurseurs érythropoïétiques relativement matures avec une capacité de prolifération limitée, et un stade tardif, caractérisé par l'émergence de précurseurs indifférenciés avec une capacité de prolifération intense. In vivo, les manifestations de cette maladie sont une splénomégalie accompagnée d'une hépatomégalie, d'une hyperplasie érythroïde dans la rate et la moelle osseuse, d'une polycytémie et de foci macroscopiques dans la rate. Les dernières étapes sont caractérisées par la présence de cellules érythroïdes immortelles et transplantables.

On a étudié l'effet du DAH sur la réplication du virus de Friend en culture de cellules.

Des cultures de cellules SC1 traitées avec du polybrène ont été infectées avec du FLV (5 x 10⁵ particules infectantes pour 2 x 10⁵ cellules). Les cellules ont ensuite été mises en culture avec diverses concentrations de DAH et l'activité de réplication du FLV a été mesurée par dosage de l'activité transcriptase inverse apparaissant dans les surnageants de milieux de culture aux 2ème, 4ème et 6ème jour après l'infection.

On observe une inhibition significative de la réplication du FLV par le DAH. L'activité transcriptase inverse est abaissée d'environ 70 % par le DAH à 0,2 mM, de 80 % à 0,5 mM et de 90 % à 1 mM.

En revanche, on observe que les doses 0,2 et 0,5 mM de DAH n'ont aucun effet cytostatique sur les cellules SC1 et qu'à 1 mM le DAH n'inhibe que modérément la division cellulaire. Ainsi, l'effet anti-FLV observé ne résulte pas indirectement d'un effet toxique du DAH sur le métabolisme cellulaire mais d'un effet direct sur la réplication du rétrovirus.

La transcriptase inverse étant la cible de divers agents anti-rétroviraux, il est intéressant de tester l'effet du DAH sur l'activité transcriptase inverse (RT) du FLV. Cet essai a été effectué en utilisant comme source de RT une préparation de FLV hautement purifié, et comme substrat du poly A-oligo dT. Les substrats ont été introduits dans le milieu réactionnel dans l'ordre suivant : DAH aux concentrations désirées, virus purifié, polyA-oligo dT, dTTP + ³HdTTP.

Les résultats montrent que le DAH n'a aucun effet sur l'activité RT aux concentrations auxquelles on observe l'effet anti-rétroviral en culture cellulaire. Le DAH, sous la forme native non métabolisée, n'est donc pas un inhibiteur de la RT.

Dans d'autres essais, le virus a été maintenu par passages successif intrapéritonéaux chez la souris DBA/2. Cet entretien a été fait à partir de la rate de l'animal tous les 21 jours. L'activité du DAH a été évaluée en comparant les durées de survie des animaux à qui est injecté le virus, avec ou sans DAH. Le traitement a commencé trois jours après l'infection virale car on considère qu'il faut ce laps de temps pour qu'il y ait des séquences du virus dans le génome des précurseurs érythroïdes de la souris infectée. Après avoir fait varier le nombre d'injections journalières et la durée du traitement pour une dose donnée, il a été établi que 3 injections de 1g/kg/dose de DAH durant les 10 premiers jours et 2 injections de 1g/kg/dose de DAH pendant les 20 jours suivants, produisent un T/C de 239 %. Les résultats sont rapportés dans le tableau suivant :

**Tableau 2**

| Substance active | Dose (g/kg de poids corporel) | jours de traitement | T/C x 100 (%) (2) | Nombre d'animaux survivants |
|---|---|---|---|---|
| DAH | 1 x 3/j (1) | J 1-30 | 239,86 | 5/10 |
| | 1 x 2/j | | | |
| DAH | 1 x 3/j | J 1-10 | 193 | 0/10 |

| | | | | |
|---|---|---|---|---|
| (1) Les animaux sont traités 3 fois par jour de J 1-10 et 2 fois par jour de J 11-30. | | | | |
| (2) T/C = rapport de la durée de survie des animaux traités (T) et de la durée de survie des animaux témoins (C). | | | | |

On notera qu'un critère d'activité T/C égal ou supérieur à 125 % est significatif.

### ACTIVITE ANTITUMORALE DU DAH

Selon l'invention, on a aussi montré que le DAH, lorsqu'il est administré dans des dosages spécifiques, tels que définis ci-après, a une action inhibitrice sur les tumeurs.

Dans la demande de brevet français précité FR-2.600.256, il est rapporté que le DAH n'a montré aucune activité in vive, après un traitement par DAH à raison de 600 mg/kg par jour pendant 5 jours, sur trois modèles tumoraux murins : leucémie 1210, lymphome RBL5 et mélanome B16, les souris ayant été inoculées avec les cellules tumorales précitées. Il a maintenant été observé, d'une part, que, dans les mêmes types d'essais conduits in vitre, l'inhibition de la croissance des cellules tumorales (IC 50 %) a lieu pour des doses de DAH commençant entre 0,8 et 1 mM par litre de milieu cellulaire, et, d'autre part, que, sur le modèle tumoral murin de la leucémie 1210 réputée difficilement guérissable, une administration de 1 g/kg de poids corporel, à raison de 4 fois par jour pendant 3 jours, conduit déjà à une prolongation très significative de la survie des animaux traités.

Dans ces mêmes conditions de dosage, le L-aspartique acide β hydroxamate est très toxique. Ainsi, l'augmentation des doses du dérivé D précité par rapport au dérivé L fait apparaître une activité du dérivé D, sans présenter la toxicité que présente le dérivé L aux mêmes dosages (la DL 50 % pour le DAH est de 10 g/kg alors que la DL 50 % pour le LAH est de 2,5 g/kg).

Les doses employées pour le traitement des souris ont varié de 0,5 g/kg à 1,5 g/kg pendant une période minimale de 3 jours pouvant s'étendre jusqu'à 10 jours.

Il s'avère cependant qu'il est possible d'employer des doses plus élevées, jusqu'à 5 g/kg et que si la dose est faible, il faut une période de traitement plus longue.

### Etude comparative in vivo du DAH et du LAH

L'activité antitumorale du DAH et du LAH a été étudiée sur le modèle de leucémie L1210. Les cellules leucémiques L1210 sont maintenues par passages intrapéritonéaux dans la souris B6D2F1/J mâle. Le nombre de cellules utilisées pour les essais thérapeutiques est de 105 cellules par souris dans 0,5 ml de PBS. La durée d'observation est de 30 jours.

Les résultats sont rapportés dans le Tableau 3 suivant :

**Tableau 3**

| Substance active | Dose (g/kg de poids corporel) | Jours de traitement | T/Cx100 (%) (2) | Nombre(3) d'animaux survivants | Evolution pondérale (4) |
|---|---|---|---|---|---|
| DAH | 1x3/j | 1-10 | 347 | 3/5 | + 1,300 |
| DAH | 1,5x4/j(1) | 1,2,3,4 | 347 | 3/5 | + 1,000 |
| DAH | 1,5x4/j | 1,2,3 | 276 | 0/5 | + 1,200 |
| DAH | 1x4/j | 1,2,3 | 171 | 0/5 | + 1,100 |
| DAH | 0,5x4/j | 1,2,3 | 113 | 0/5 | + 1,400 |
| LAH | 1,5x4/j | 1 | 50 | 0/5 | - |
| LAH | 1x4/j | 1,2 | 65 | 0/5 | - |

| | | | | | |
|---|---|---|---|---|---|
| (1) Les animaux sont traités 4 fois par jour. | | | | | |
| (2) T/C = rapport de la durée de survie des animaux traités (T) et de la durée de survie des animaux témoins (C). | | | | | |
| (3) à 30 jours. | | | | | |
| (4) Evolution pondérale (en grammes) de J1 - J5. | | | | | |

On constate que le DAH manifeste une activité antitumorale significative sur ce modèle pour une administration à une dose supérieure ou égale à 1 g/kg, 3 ou 4 fois par jour. En revanche, pour le LAH, le même protocole n'est pas applicable en raison de la toxicité, et même en utilisant un protocole plus léger, on n'a pas observé d'activité antitumorale, mais toujours une toxicité très importante (mort des animaux traités avec le LAH avant le groupe témoin).

L'activité antitumorale du DAH a été aussi étudiée sur deux autres modèles murins : le lymphome L5178Y et la leucémie P388.

Les cellules de lymphome murin (L5178Y) sont maintenues par passages intrapéritonéaux successifs chez la souris DBA/2 et injectées, pour les essais, à la dose de 104 cellules par souris.

Les cellules de la leucémie P388 sont maintenues par passage intrapéritonéaux chez la souris DBA/2 et injectées, pour les essais, à la dose de 105 cellules par souris sur la souris B6D2F1.

Les résultats sont rapportés dans le tableau suivant :

| Lymphome L5178Y | | | | |
|---|---|---|---|---|
| Substance active | Dose (g/kg de poids corporel | Jours de traitement | T/Cx100 (%) | Nombre d'animaux survivants* |
| DAH | 1 x 4/j | 1,2,3,4,5 | 576 | 5/5 |
| DAH | 1 x 3/j | 1,2,3,4,5 | 576 | 5/5 |

| | | | | |
|---|---|---|---|---|
| * à 120 jours. | | | | |

| Leucémie P388 | | | | |
|---|---|---|---|---|
| Substance active | Dose (g/kg de poids corporel | Jours de traitement | T/Cx100 (%) | Nombre d'animaux survivants* |
| DAH | 1,5 x 4/j | 1,2,3,4,5 | 190 | 0/5 |
| DAH | 1,5 x 4/j | 1,2,3 | 155 | 0/5 |

| | | | | |
|---|---|---|---|---|
| * à 30 jours. | | | | |

Dans la description qui suit, on a donné des indications plus complètes sur l'utilisation du DAH comme médicament.

### Phamacologie pré-clinique

Le DAH a été dosé dans les sérums et les urines de souris B6D2F1 âgées de 8 semaines, ayant reçu une injection intrapéritonéale de DAH de 3 g/kg. Le DAH a été dosé par spectrophotométrie par mise en évidence du complexe Fe3+- hydroxamate à 550 nm.

Après injection intrapéritonéale de DAH on a observé un pic plasmatique moyen de 6 mg/ml à 20 minutes. La décroissance de cette concentration s'est faite avec une demi-vie de 35 minutes. Le pic urinaire a été observé à 250 minutes après l'injection avec une valeur de 220 mg/ml. L'élimination urinaire s'est prolongée sur 24 heures. Le DAH éliminé par voie urinaire n'a subi aucune modification structurale. Le DAH agit donc sous sa forme native, sans avoir subi de métabolisme.

### Etude toxicologique

### Toxicité chez la souris :

La toxicité du DAH par voie parentérale a été évaluée chez la souris B6D2F1 âgée de six semaines et pesant entre 20 et 22 g. Pour chaque niveau de dose, on a utilisé treize mâles et treize femelles avec une période d'observation de 14 jours. La dose léthale (DL) a été évaluée 24 heures après l'injection de DAH puis à 14 jours. Dans ces conditions, on a pu montrer que la DL 10 (10 % de mortalité) était observée pour des posologies de 8,5-9 g/kg, la DL 50 correspondait à 11-12 g/kg et la DL 90 à 14-15 g/kg. Il n'a pas été observé de différence significative de mortalité à 24 heures et à 14 jours pour un niveau de dose donné.

La tolérance à l'administration prolongée du DAH a été évaluée sur un groupe de 10 souris traitées pendant 30 jours à une injection quotidienne de 2 g/kg, et un groupe de 10 souris recevant pendant la même durée deux injections de 1 g/kg par jour. La tolérance a été excellente, sans signe clinique de toxicité, sans décès et avec un poids corporel restant dans un intervalle de +/- 10 % par rapport à un groupe contrôle de même âge et de même poids initial.

### Toxicité chez le chien :

En suivant les recommandations du National Cancer Institute (NCI), on a déterminé : a) la dose maximale non toxique, b) la dose toxique faible, c) la dose maximale toxique non léthale, d) la dose léthale.

Ces études ont été réalisées sur des chiens Beagle âgés de six à sept mois et pesant 6,5 à 7,5 kg. Pour mettre en évidence les effets toxiques du DAH, on a mesuré des paramètres cliniques, morphologiques, biochimiques et hématologiques.

L'évaluation de la fréquence cardiaque, l'électrocardiogramme, la pression artérielle (mesurée par cathétérisme intra-artériel), et la fréquence respiratoire ont été mesurées chez des chiens anesthésiés par l'alphachloralose. Chez des animaux ayant reçu une administration intraveineuse de DAH variant entre 1 et 5 g/kg, aucune modification de ces paramètres n'a été observée.

Effets toxiques d'apparition immédiate : l'injection intraveineuse de DAH à des doses variant de 1 à 10 g/kg, (en solution à 10 %) a provoqué des vomissements pendant la durée d'administration du produit. Ces vomissements ont disparu dès la fin de la perfusion et s'accompagnent d'une anorexie qui peut se prolonger jusqu'à 24 heures, selon l'importance de la dose administrée. Aucun autre symptome d'intolérance n'a été observé en cours d'administration du DAH.

Effets toxiques d'apparition précoce : toxicité hématologique. Une toxicité sur la lignée plaquettaire a été constatée à partir de la dose de 5 g/kg, avec un nadir de 39 x 109 plaquettes/l au 8ème jour après l'injection de DAH, et un retour au niveau pré-traitement sept à dix jours plus tard. A la dose de 9 g/kg la thrombopénie était plus profonde avec un nadir à 17 x 109 plaquettes/l vingt deux jours après l'injection et une récupération partielle (56 x 10⁹/l) deux mois plus tard. Aucune toxicité sur les lignées granulo-monocytaires ni érythrocytaires n'a été constatée, y compris aux fortes doses (9 g/kg).

Aucune toxicité de la fonction rénale ou hépatique n'a été constatée aux doses décrites ci-dessus. Un chien ayant reçu une dose de plus de 10 g/kg a présenté une précipitation des cristaux de DAH dans les tubules rénaux, complication qui a pu être évitée par la suite par une préhydratation avec du sérum physiologique.

L'administration réitérée de DAH par voie intraveineuse sur deux semaines, avec cinq injections hebdomadaires de 1 g/kg n'a mis en évidence aucun signe d'intolérance autre que des vomissements pendant la durée d'administration du produit. Les examens biologiques n'ont montré aucune perturbation métabolique, hématologique ni de l'hémostase.

L'ensemble de ces résultats permet de conclure que la dose maximale non toxique se situe entre 2,5 et 3 g/kg, que la dose toxique faible est de 5 g/kg et que la dose toxique maximale non léthale est de l'ordre de 9 g/kg. Ceci démontre la faible toxicité du DAH.

Le DAH peut être administré par voie intraveineuse continue à des malades atteints du SIDA. A cet effet, le DAH en poudre est mis en solution physiologique, par exemple à une concentration voisine de 10 %. Chaque flacon contient 10 g de DAH pour une dose unique. Le DAH est alors administré sur 24 h en perfusion continue par voie veineuse périphérique.

Les études pré-cliniques présentées ci-dessus fournissent une valeur de la DL 10 de 8,5 g/kg. En tenant compte du facteur de correction souris/homme, la dose initiale chez l'homme serait donc de 1,2 g/m² de surface corporelle.

Compte-tenu des études d'activité effectuées chez l'animal, on peut pratiquer une administration en perfusion veineuse continue sur une période de 15 jours, après que les malades aient reçu une dose unitaire égale au 1/24ème de la dose quotidienne, en une perfusion d'une heure, la veille de traitement par perfusion continue. Ce protocole est voisin de celui qui a été employé dans le cas de l'azidothymidine (Yarchoan et al., Lancet, 1986, 575-580). L'augmentation des doses peut être réalisée dans le cadre de la surveillance médicale.

La description qui précède illustre l'intérêt pratique du DAH dans le traitement du SIDA. D'autre part, l'utilisation du DAH a été illustrée dans la description à l'encontre spécifiquement du virus HIV, mais cette utilisation concerne le traitement de toutes les affections liées au SIDA.

## Revendications (Revendications pour l'(les) Etat(s) contractant(s) suivant(s): AT, BE, CH, LI, DE, DK, FR, GB, IT, LU, NL, SE)

1. Médicament, caractérisé en ce qu'il comprend du D-acide aspartique β monohydroxamate (DAH), comme agent thérapeutique, dans une quantité thérapeutiquement active, à l'exclusion de toute composition médicamenteuse dans laquelle le DAH est, soit présent dans une quantité subthérapeutique, soit en combinaison avec la L-asparaginase.

2. Médicament selon la revendication 1, caractérisé en ce que le DAH est le seul agent thérapeutique.

3. Médicament selon la revendication 1, caractérisé en ce qu'il comprend un support pharmaceutiquement acceptable, adapté à une administration par voie orale.

4. Médicament selon la revendication 1, caractérisé en ce qu'il est sous une forme adaptée à une administration parentérale, notamment par voie veineuse périphérique.

5. Utilisation du D-acide aspartique β monohydroxamate (DAH) pour l'obtention d'un médicament, à l'exclusion de toute utilisation du DAH pour l'obtention d'une composition médicamenteuse dans laquelle le DAH est, soit présent dans une quantité subthérapeutique, soit en combinaison avec la L-asparaginase.

6. Utilisation selon la revendication 5, pour l'obtention d'un médicament anti-tumoral.

7. Utilisation selon la revendication 5, pour l'obtention d'un médicament anti-viral, plus particulièrement un médicament anti-rétroviral, et notamment comme agent contre les virus du SIDA.

8. Utilisation d'une composition comprenant le D-acide aspartique β monohydroxamate (DAH), pour l'obtention d'un médicament, à l'exclusion de l'utilisation de toute composition dans laquelle le DAH est, soit présent dans une quantité subthérapeutique, soit en combinaison avec la L-asparaginase.

9. Utilisation selon la revendication 8, pour l'obtention d'un médicament anti-tumoral.

10. Utilisation selon la revendication 8, pour l'obtention d'un médicament anti-viral, plus particulièrement un médicament anti-rétroviral, et notamment comme agent contre les virus du SIDA.

11. Utilisation du DAH ou d'une composition comprenant du DAH, à i'exclusion de l'utilisation de toute composition dans laquelle le DAH est, soit présent dans une quantité subthérapeutique, soit en combinaison avec la L-asparaginase, pour l'obtention d'un médicament antirétroviral, et notamment comme agent contre les virus du SIDA.

12. Utilisation selon l'une quelconque des revendications 5 à 11, pour l'obtention d'un médicament sous une forme adaptée à une administration orale.

13. Utilisation selon l'une quelconque des revendications 5 à 11, pour l'obtention d'un médicament sous une forme adaptée à une administration parentérale, notamment par la voie veineuse périphérique.

## Revendications (Revendications pour l'(les) Etat(s) contractant(s) suivant(s): ES)

1. Procédé de préparation d'un médicament, caractérisé en ce qu'on utilise du D-acide aspartique β monohydroxamate (DAH), à titre d'agent thérapeutique et dans une quantité thérapeutiquement active, à l'exclusion de toute composition comprenant du DAH, soit présent dans une quantité subthérapeutique, soit en combinaison avec la L-asparaginase.

2. Procédé de préparation, selon la revendication 1, caractérisé en ce qu'on met en oeuvre du DAH, comme seul agent thérapeutique.

3. Procédé de préparation selon la revendication 1, caractérisé en ce qu'on met en oeuvre un support pharmaceutiquement acceptable, adapté à une administration par voie orale.

4. Procédé de préparation selon la revendication 1, caractérisé en ce qu'on met en oeuvre une forme adaptée à une administration parentérale, notamment par voie veineuse périphérique.

5. Utilisation du D-acide aspartique β monohydroxamate (DAH) pour l'obtention d'un médicament, à l'exclusion de toute utilisation du DAH pour l'obtention d'une composition médicamenteuse dans laquelle le DAH est, soit présent dans une quantité subthérapeutique, soit en combinaison avec la L-asparaginase.

6. Utilisation selon la revendication 5, pour l'obtention d'un médicament anti-tumoral.

7. Utilisation selon la revendication 5, pour l'obtention d'un médicament anti-viral, plus particulièrement un médicament anti-rétroviral, et notamment comme agent contre les virus du SIDA.

8. Utilisation d'une composition comprenant le D-acide aspartique β monohydroxamate (DAH), pour l'obtention d'un médicament, à l'exclusion de l'utilisation de toute composition dans laquelle le DAH est, soit présent dans une quantité subthérapeutique, soit en combinaison avec la L-asparaginase.

9. Utilisation selon la revendication 8, pour l'obtention d'un médicament anti-tumoral.

10. Utilisation selon la revendication 8, pour l'obtention d'un médicament anti-viral, plus particulièrement un médicament anti-rétroviral, et notamment comme agent contre les virus du SIDA.

11. Utilisation du DAH ou d'une composition comprenant du DAH, à l'exclusion de l'utilisation de toute composition dans laquelle le DAH est, soit présent dans une quantité subthérapeutique, soit en combinaison avec la L-asparaginase, pour l'obtention d'un médicament antirétroviral, et notamment comme agent contre les virus du SIDA.

12. Utilisation selon l'une quelconque des revendications 5 à 11, pour l'obtention d'un médicament sous une forme adaptée à une administration orale.

13. Utilisation selon l'une quelconque des revendications 5 à 11, pour l'obtention d'un médicament sous une forme adaptée à une administration parentérale, notamment par la voie veineuse périphérique.

## Claims (Claims for the following Contracting State(s): AT, BE, CH, LI, DE, DK, FR, GB, IT, LU, NL, SE)

1. Medicament, characterized in that it comprises D-aspartic acid β-monohydroxamate (DAH) as therapeutic agent, in a therapeutically active quantity, excluding any medicinal composition in which DAH is either present in a subtherapeutic quantity or in combination with L-asparaginase.

2. Medicament according to Claim 1, characterized in that DAH is the sole therapeutic agent.

3. Medicament according to Claim 1, characterized in that it comprises a pharmaceutically acceptable vehicle suitable for oral administration.

4. Medicament according to Claim 1, characterized in that it is in a form suitable for parenteral administration, in particular via the peripheral venous route.

5. Use of D-aspartic acid β-monohydroxamate (DAH) for obtaining a medicament, excluding any use of DAH for obtaining a medicinal composition in which DAH is either present in a subtherapeutic quantity or in combination with L-asparaginase.

6. Use according to Claim 5, for obtaining an antitumour medicament.

7. Use according to Claim 5, for obtaining an antiviral medicament, more especially an antiretroviral medicament, and in particular as an agent against AIDS viruses.

8. Use of a composition comprising D-aspartic acid β-monohydroxamate (DAH) for obtaining a medicament, excluding the use of any composition in which DAH is either present in a subtherapeutic quantity or in combination with L-asparaginase.

9. Use according to Claim 8, for obtaining an antitumour medicament.

10. Use according to Claim 8, for obtaining an antiviral medicament, more especially an antiretroviral medicament, and in particular as an agent against AIDS viruses.

11. Use of DAH or of a composition comprising DAH, excluding the use of any composition in which DAH is either present in a subtherapeutic quantity or in combination with L-asparaginase, for obtaining an antiretroviral medicament, and in particular as an agent against AIDS viruses.

12. Use according to any one of Claims 5 to 11, for obtaining a medicament in a form suitable for oral administration.

13. Use according to any one of Claims 5 to 11, for obtaining a medicament in a form suitable for parenteral administration, in particular via the peripheral venous route.

## Claims (Claims for the following Contracting State(s): ES)

1. Method for preparing a medicament, characterized in that D-aspartic acid β-monohydroxamate (DAH) is used as therapeutic agent and in a therapeutically active quantity, excluding any composition comprising DAH either present in a subtherapeutic quantity or in combination with L-asparaginase.

2. Preparation method according to Claim 1, characterized in that DAH is employed as sole therapeutic agent.

3. Preparation method according to Claim 1, characterized in that a pharmaceutically acceptable vehicle suitable for oral administration is employed.

4. Preparation method according to Claim 1, characterized in that a form suitable for parenteral administration, in particular via the peripheral venous route, is employed.

5. Use of D-aspartic acid β-monohydroxamate (DAH) for obtaining a medicament, excluding any use of DAH for obtaining a medicinal composition in which DAH is either present in a subtherapeutic quantity or in combination with L-asparaginase.

6. Use according to Claim 5, for obtaining an antitumour medicament.

7. Use according to Claim 5, for obtaining an antiviral medicament, more especially an antiretroviral medicament, and in particular as an agent against AIDS viruses.

8. Use of a composition comprising D-aspartic acid β-monohydroxamate (DAH) for obtaining a medicament, excluding the use of any composition in which DAH is either present in a subtherapeutic quantity or in combination with L-asparaginase.

9. Use according to Claim 8, for obtaining an antitumour medicament.

10. Use according to Claim 8, for obtaining an antiviral medicament, more especially an antiretroviral medicament, and in particular as an agent against AIDS viruses.

11. Use of DAH or of a composition comprising DAH, excluding the use of any composition in which DAH is either present in a subtherapeutic quantity or in combination with L-asparaginase, for obtaining an antiretroviral medicament, and in particular as an agent against AIDS viruses.

12. Use according to any one of Claims 5 to 11, for obtaining a medicament in a form suitable for oral administration.

13. Use according to any one of Claims 5 to 11, for obtaining a medicament in a form suitable for parenteral administration, in particular via the peripheral venous route.

## Patentansprüche (Patentansprüche für folgende(n) Vertragsstaat(en): AT, BE, CH, LI, DE, DK, FR, GB, IT, LU, NL, SE)

1. Arzneimittel, dadurch gekennzeichnet, daß es als therapeutischen Wirkstoff D-Asparagino-β-monohydroxamsäure (DAH) in einer therapeutisch aktiven Menge enthält, unter Ausschluß jeglicher Arzneimittelzusammensetzung, in der die DAH entweder in einer subtherapeutischen Menge oder in Kombination mit L-Asparaginase vorhanden ist.

2. Arzneimittel nach Anspruch 1, dadurch gekennzeichnet, daß die DAH der einzige therapeutische Wirkstoff ist.

3. Arzneimittel nach Anspruch 1, dadurch gekennzeichnet, daß es einen pharmazeutisch verträglichen Träger enthält, der für eine orale Verabreichung geeignet ist.

4. Arzneimittel nach Anspruch 1, dadurch gekennzeichnet, daß es in einer für eine parenterale Verabreichung, insbesondere für den peripher venösen Weg, geeigneten Form vorliegt.

5. Verwendung von D-Asparagino-β-monohydroxamsäure (DAH) zum Herstellen eines Arzneimittels, unter Ausschluß jeglicher Verwendung der DAH zum Herstellen einer Arzneimittelzusammensetzung, in der die DAH entweder in einer subtherapeutischen Menge oder in Kombination mit L-Asparaginase vorhanden ist.

6. Verwendung nach Anspruch 5 zum Herstellen eines Arzneimittels gegen Tumore.

7. Verwendung nach Anspruch 5 zum Herstellen eines Arzneimittels gegen Viren, insbesondere eines Arzneimittels gegen Retroviren, und insbesondere als Wirkstoff gegen HIV(SIDA)-Viren.

8. Verwendung einer Zusammensetzung, die D-Asparagino-β-monohydroxamsäure (DAH) enthält, zum Herstellen eines Arzneimittels, unter Ausschluß jeglicher Verwendung von Zusammensetzungen, in denen die DAH entweder in einer subtherapeutischen Menge oder in Kombination mit L-Asparaginase vorhanden ist.

9. Verwendung nach Anspruch 8, um ein Arzneimittel gegen Tumore zu erhalten.

10. Verwendung nach Anspruch 8, um ein Arzneimittel gegen Viren, insbesondere ein Arzneimittel gegen Retroviren zu erhalten, und insbesondere als Wirkstoff gegen HIV(SIDA)-Viren.

11. Verwendung von DAH oder einer Zusammensetzung, die DAH enthält, mit der Ausnahme der Verwendung von jeglichen Zusammensetzungen, in denen die DAH entweder in einer subtherapeutischen Menge oder in Kombination mit L-Asparaginase vorhanden ist, um ein Arzneimittel gegen Retroviren zu erhalten, und insbesondere als Wirkstoff gegen HIV(SIDA)-Viren.

12. Verwendung nach einem der Ansprüche 5 bis 11, bei der ein Arzneimittel in einer Form erhalten wird, die für die orale Verabreichung geeignet ist.

13. Verwendung nach einem der Ansprüche 5 bis 11, bei der ein Arzneimittel in einer Form erhalten wird, die für eine parenterale Verabreichung, insbesondere auf peripher venösem Weg, geeignet ist.

## Patentansprüche (Patentansprüche für folgende(n) Vertragsstaat(en): ES)

1. Verfahren zum Herstellen eines Arzneimittels, dadurch gekennzeichnet, daß man D-Asparagino-β-monohydroxamsäure (DAH) als therapeutischen Wirkstoff und in einer therapeutisch aktiven Menge verwendet, unter Ausschluß jeglicher Zusammensetzung, in der die DAH entweder in subtherapeutischer Menge oder in Kombination mit L-Asparaginase vorhanden ist.

2. Herstellungsverfahren nach Anspruch 1, dadurch gekennzeichnet, daß die DAH als einziger therapeutischer Wirkstoff eingesetzt wird.

3. Herstellungsverfahren nach Anspruch 1, dadurch gekennzeichnet, daß ein pharmazeutisch verträglicher Träger eingesetzt wird, der für eine orale Verabreichung geeignet ist.

4. Herstellungsverfahren nach Anspruch 1, dadurch gekennzeichnet, daß eine Form hergestellt wird, die für eine parenterale Verabreichung, insbesondere für den peripher venösen Weg geeignet ist.

5. Verwendung von D-Asparagino-β-monohydroxamsäure (DAH) zum Herstellen eines Arzneimittels, unter Ausschluß jeglicher Verwendung der DAH zum Herstellen einer Arzneimittelzusammensetzung, in der die DAH entweder in einer subtherapeutischen Menge oder in Kombination mit L-Asparaginase vorhanden ist.

6. Verwendung nach Anspruch 5 zum Herstellen eines Arzneimittels gegen Tumore.

7. Verwendung nach Anspruch 5 zum Herstellen eines Arzneimittels gegen Viren, insbesondere eines Arzneimittels gegen Retroviren, und insbesondere als Wirkstoff gegen HIV(SIDA)-Viren.

8. Verwendung einer Zusammensetzung, die D-Asparagino-β-monohydroxamsäure (DAH) enthält, zum Herstellen eines Arzneimittels, unter Ausschluß jeglicher Verwendung von Zusammensetzungen, in denen die DAH entweder in einer subtherapeutischen Menge oder in Kombination mit L-Asparaginase vorhanden ist.

9. Verwendung nach Anspruch 8, um ein Arzneimittel gegen Tumore zu erhalten.

10. Verwendung nach Anspruch 8, um ein Arzneimittel gegen Viren, insbesondere ein Arzneimittel gegen Retroviren zu erhalten, und insbesondere als Wirkstoff gegen HIV(SIDA)-Viren.

11. Verwendung von DAH oder einer Zusammensetzung, die DAH enthält, mit der Ausnahme der Verwendung von jeglichen Zusammensetzungen, in denen die DAH entweder in einer subtherapeutischen Menge oder in Kombination mit L-Asparaginase vorhanden ist, um ein Arzneimittel gegen Retroviren zu erhalten, und insbesondere als Wirkstoff gegen HIV(SIDA)-Viren.

12. Verwendung nach einem der Ansprüche 5 bis 11, bei der ein Arzneimittel in einer Form erhalten wird, die für die orale Verabreichung geeignet ist.

13. Verwendung nach einem der Ansprüche 5 bis 11, bei der ein Arzneimittel in einer Form erhalten wird, die für eine parenterale Verabreichung, insbesondere auf peripher venösem Weg, geeignet ist.
